# EUROPEAN PATENT APPLICATION

(11) **EP 2 042 487 A2**
(43) Date of publication of application: **01.04.2009**
(21) Application number: 08016538.4
(22) Date of filing: 19.09.2008
(51) Int. Cl.: C07C 381/12

(54) **Production method of sulfonium salt compound**

(30) Priority: 28.09.2007 JP 2007256374
(71) Applicant: FUJIFILM Corporation, Minato-ku Tokyo (JP)
(72) Inventor: Ukai, Toshinao, Odawara-shi, Kanagawa (JP); Mori, Hideto, Odawara-shi, Kanagawa (JP)
(74) Representative: HOFFMANN EITLE

(57) **Abstract**

A production method of a sulfonium salt compound represented by the following general formula (1): 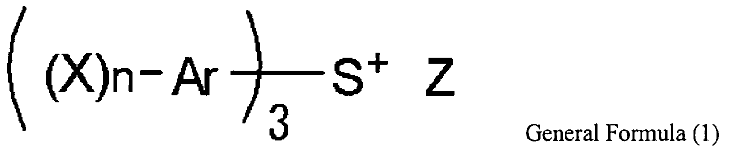 wherein Ar represents an aromatic compound residue, X represents a halogen atom, which is a substituent on the aromatic compound residue, n represents an integer of from 1 to 7, a plurality of each of Ar, X and n may be the same or different, and Z represents a counteranion other than a halide ion,
the method comprising:
reacting an aryl halide compound with a compound represented by the following general formula (2) in the presence of a Lewis acid 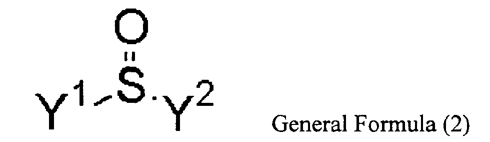 wherein Y¹ and Y² each independently represent a halogen atom, an alkoxy group or an aryloxy group;
terminating the reaction by water in an after-treatment step of the reaction;
isolating a water layer from the reaction mixture that has been separated into two layers;
extraction-treating the water layer by an organic solvent to remove a by-product;
adding a compound represented by the following general formula (3) to the extraction-treated water layer to precipitate the sulfonium salt compound represented by the general formula (1)

**M-Z** General Formula (3)

wherein Z is as defined above, and M represents a proton or an alkali metal cation; and
isolating the precipitated sulfonium salt compound by solid-liquid separation.

## Description

### TECHNICAL FIELD

The present invention relates to a production method of a sulfonium salt compound that is useful as an acid generating agent for resists, a photocation polymerization initiator or a photoradical polymerization initiator.

### BACKGROUND ART

As a conventional negative-type photosensitive planographic printing plate precursor, those having a lipophilic polymerizable composition on a hydrophilic support have been widely used. The polymerizable composition is curable upon light exposure or heating, and in an image recording method using such a planographic printing plate precursor, an intended image is obtained usually by curing the exposed area by mask exposure via a litho film or the like, and dissolving and removing the non-image area (unexposed area) using an alkali developer or the like.

In recent years, digitization technologies in which image information is electronically processed, accumulated and output using a computer or the like have been widely used, and CTP (Computer to Plate) technologies in which a printing plate is produced directly from a computer using laser light without using a litho film as mentioned above have been established. For example, a negative-type CTP printing plate, which is a chemical amplification type plate comprising, as recording layer components of the planographic printing plate precursor, an acid generating agent that generates an acid by light or heat and a polymerizable compound that initiates and promotes a polymerization reaction using the acid generated from the acid generating agent as an initiator (e.g., Japanese Patent Application Laid-Open (JP-A) No. 7-20629), and a negative-type CTP printing plate, which comprises, as recording layer components, a radical polymerization initiator that generates a radical by heat or light and a polymerizable compound that initiates and promotes a polymerization reaction using the radical generated from the radical polymerization initiator as an initiator (e.g., JP-A No. 2001-343742) have been developed.

Furthermore, in the field of semiconductors, light sources for irradiation apparatuses used in fine processing, particularly in lithography, have come to have shorter wavelengths in accordance with integration of semiconductor elements with high density in recent years. Under such circumstances, chemical amplification-type resist compositions comprising an acid generating agent in a photosensitive composition to generate an acid from the acid generating agent upon light exposure and form an image by the acid, have been commonly used in order to allow formation of fine images by laser. As the acid generating agent used for the chemical amplification-type resist composition, sulfonium salts, iodonium salts, diazodisulfone compounds and the like have been studied. Furthermore, onium salts such as sulfonium salts, iodonium salts and diazonium salts have also been used as a cation polymerization initiator or a radical polymerization initiator (e.g., JP-ANo. 2001-343742).

As an acid generating agent or a radical polymerization initiator for the CTP printing plates, or an acid generating agent for the resist composition, onium salts such as iodonium salts, diazonium salts and sulfonium salts are preferably used. Among these, sulfonium salts have excellent balance of stability and reactivity as an acid generating agent or a radical generating agent. Specifically, particularly preferable sulfonium salts include triaryl sulfonium salts.

As synthesis methods of triaryl sulfonium salts, for example, a method comprising reacting a diarylsulfoxide and a Grignard reaction agent (e.g., J. Am. Chem. Soc., Vol. 73, page 1965 (1951), and J. Am. Chem. Soc., Vol. 112, page 6004 (1990)), a method comprising reacting a diarylsulfoxide and an aromatic hydrocarbon in the presence of aluminum chloride (e.g., J. Org. Chem., Vol. 33, page 2671 (1968)), a method comprising reacting a diarylsulfide and a diaryliodonium salt (e.g., J. Org. Chem., Vol. 43, page 3055 (1978)), and the like have been known.

Meanwhile, a method comprising reacting thionyl chloride and a Grignard reaction agent in a specific solvent has been disclosed (e.g., JP-A No. 2004-315430). Furthermore, it has been known that a tris(p-hydroxyphenyl)sulfonium salt is directly obtained by reacting phenol and thionyl chloride in the presence of aluminum chloride (e.g., J. Am. Chem. Soc., Vol. 82, page 5359 (1960)).

### DISCLOSURE OF INVENTION

In the methods described in J. Am. Chem. Soc., Vol. 73, page 1965 (1951), J. Am. Chem. Soc., Vol. 112, page 6004 (1990), and J. Org. Chem., Vol. 33, page 2671 (1968), a diarylsulfide or a diarylsulfoxide is used as a starting raw material. For example, in order to synthesize a triaryl sulfonium salt having substituents on the aryl group moieties, a corresponding diarylsulfide or diarylsulfoxide having substituents on the aryl group moieties in advance is necessary. However, such diarylsulfides and diarylsulfoxides are not necessarily easy to obtain, and steps and cost for synthesizing these compounds in advance are often required.

The method of JP-A No. 2004-315430 is excellent in that the method may be started from thionyl chloride which is inexpensive and readily available, but on an industrial scale, it is not necessarily easy to stably and safely prepare a Grignard reaction agent, which is highly reactive and water-reactive. Specifically, synthesis of a sulfonium salt in which an aryl group is substituted by a substituent, specifically by a halogen atom, lacks versatility since preparation of a Grignard reaction agent is difficult, a reaction intermediate (sulfoxide) easily precipitates, the yield of the objective substance is significantly decreased, and the like.

In the method of J. Org. Chem., Vol. 43, page 3055 (1978), use of a Grignard reaction agent is not required, but the case where this reaction is applicable is limited to the case where phenol or the like which is highly reactive is the substrate, and the article does not describe an aryl compound with low reactivity which has a halogen atom or the like as a substituent.

Furthermore, in the above-mentioned production method, a sulfonium salt is first generated in the form having a halide ion as a counterion. However, since the main material of a substrate for a printing material is aluminum, a step of exchanging the halide ion with another counterion so as to avoid corrosion is additionally required in order to use the sulfonium salt compound in the technical field, and therefore, multiple operations such as solid-liquid separation and washing are required.
As mentioned above, the conventionally-reported methods are not necessarily advantageous methods in view of versatility, availability of raw materials, safety, cost, required time and the like, and there has been a strong demand for a technology that allows production of a large amount of a sulfonium salt compound by easy operation.

Therefore, an object of the present invention is to provide a production method of a sulfonium salt compound that is useful as an acid generating agent for resists, a photocation polymerization initiator or a photoradical polymerization initiator, which may be carried out economically on an industrial scale, and is excellent in versatility, safe and inexpensive.

The inventors have conducted intensive studies on a production method of a sulfonium salt compound in view of the above-mentioned circumstances. As a result, they have confirmed that the above-mentioned problems may be solved by the following novel means, and completed the present invention.

<1> A production method of a sulfonium salt compound represented by the following general formula (1): 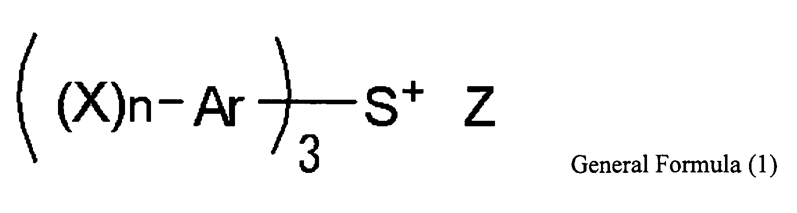 wherein Ar represents an aromatic compound residue, X represents a halogen atom, which is a substituent on the aromatic compound residue, n represents an integer of from 1 to 7, a plurality of each of Ar, X and n may be the same or different, and Z represents a counteranion other than a halide ion,
the method comprising:
reacting an aryl halide compound with a compound represented by the following general formula (2) in the presence of a Lewis acid 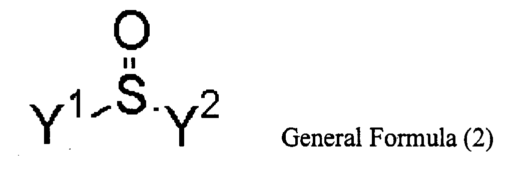 wherein Y¹ and Y² each independently represent a halogen atom, an alkoxy group or an aryloxy group;
terminating the reaction by water in an after-treatment step of the reaction;
isolating a water layer from the reaction mixture that has been separated into two layers;
extraction-treating the water layer by an organic solvent to remove a by-product;
adding a compound represented by the following general formula (3) to the extraction-treated water layer to precipitate the sulfonium salt compound represented by the general formula (1)

   **M-Z** General Formula (3)

   wherein Z is as defined above, and M represents a proton or an alkali metal cation; and
isolating the precipitated sulfonium salt compound by solid-liquid separation.

<2> The production method of <1>, wherein Ar in the general formula (1) is a benzene ring residue which may further have a substituent other than X (wherein X is as defined in <1>).

<3> The production method of <1> or <2>, wherein Y¹ and Y² in the general formula (2) are each independently a halogen atom, a methoxy group or an ethoxy group.

<4> The production method of any one of <1> to <3>, wherein the Lewis acid is selected from the group consisting of zinc chloride, aluminum chloride, aluminum bromide, antimony (V) chloride, iron (II) chloride, iron (III) chloride, titanium (IV) chloride, boron trifluoride, tin (IV) chloride and bismuth (III) chloride.

<5> The production method of any one of <1> to <4>, wherein the organic solvent for extraction-treating the water layer comprises an organic solvent selected from the group consisting of an aromatic hydrocarbon solvent, an aliphatic hydrocarbon solvent, an ether solvent and an ester solvent.

<6> The production method of any one of <1> to <5>, wherein the precipitated sulfonium salt compound is crystals.

According to the invention, various sulfonium salt compounds that are useful as an acid generating agent for resists, a photocation polymerization initiator or a photoradical polymerization initiator may be produced economically, safely and inexpensively on an industrial scale.

### BEST MODE FOR CARRYING OUT THE INVENTION

Firstly, an aryl halide compound that may be used in the present invention is explained. The aryl halide compound is not specifically limited so long as it is a compound having an aromatic ring with a halogen atom bonded to the aromatic ring as a substituent and at least one hydrogen atom bonded to the aromatic ring. Although one kind of the aryl halide compound may be used solely, or two or more kinds thereof may be used in combination, it is preferable to use one kind of the aryl halide compound. The aromatic ring in the aryl halide compound may be a monocycle or a fused ring consisting of two or more aromatic rings, and an aromatic hydrocarbon ring containing only carbon atoms are preferable. Specific examples include a benzene ring, a naphthalene ring and an anthracene ring, a benzene ring and a naphthalene ring are more preferable, and a benzene ring is further more preferable. Examples of the halogen atom in the aryl halide compound include a fluorine atom, a chlorine atom, a bromine atom and an iodine atom, of which a fluorine atom, a chlorine atom and a bromine atom are preferable.
The aryl halide compound may have a substituent other than a halogen atom on the aromatic ring. The substituent is not specifically limited so long as it does not interfere the reaction under the condition for production of the invention explained below and does not cause any side reaction, and preferable examples include branched or non-branched alkyl groups having 1 to 12 carbon atoms. The above-mentioned substituent may further be substituted where possible, and may have a ring structure.

Examples of the specific aryl halide compound include fluorobenzene, chlorobenzene, bromobenzene, iodobenzene, 1,2-difluorobenzene, 1-chloro-2-fluorobenzene, 1,2-dichlorobenzene, 1-bromo-2-chlorobenzene, 2-fluorotoluene, 2-chlorotoluene, 2-bromotoluene, 1-bromo-2-ethylbenzene, 1,3-difluorobenzene, 1,3-dichlorobenzene, 1,3-dibromobenzene, 1,4-difluorobenzene, 1,4-dichlorobenzene, 1,4-dibromobenzene, 3-fluorotoluene, 3-chlorotoluene, 3-bromotoluene, 4-fluorotoluene, 4-chlorotoluene, 4-bromotoluene, 1,3,5-trichlorobenzene, 1-chloronaphthalene, 1-bromonaphthalene, 9-chloroanthracene and 8-bromoanthracene.

Among these, fluorobenzene, chlorobenzene, bromobenzene, 1,2-dichlorobenzene, 1,4-dichlorobenzene are preferable, and fluorobenzene, chlorobenzene and 1,2-dichlorobenzene are more preferable, in view of availability of raw materials, reaction yield and the like.

Secondly, the sulfonium salt compound represented by the general formula (1) is explained.

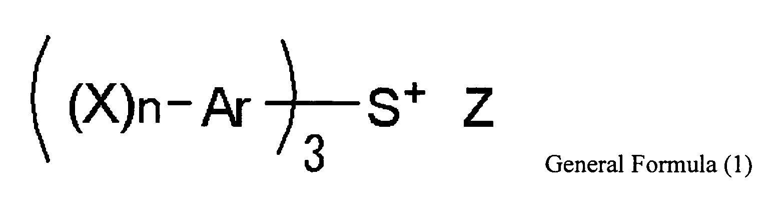

In the general formula (1), Ar represents an aromatic compound residue. The aromatic ring of the aromatic compound may be a monocycle or a fused ring consisting of two or more aromatic rings, and an aromatic hydrocarbon ring comprising only carbon atoms is preferable. Specific examples include a benzene ring, a naphthalene ring and an anthracene ring, a benzene ring and a naphthalene ring are more preferable, and a benzene ring is further more preferable.

X represents a halogen atom, which is a substituent on the aromatic compound residue. Examples of the halogen atom include a fluorine atom, a chlorine atom, a bromine atom and an iodine atom, of which a fluorine atom, a chlorine atom and a bromine atom are preferable.

n represents an integer of 1 to 7. More specifically, n is the number of halogen atoms which are substituents on the aromatic compound residue represented by Ar, and the maximum value thereof is a value obtained by subtracting the number of the substitution of the sulfur atom, 1, from the number of the substitutable positions on the aromatic ring. in is preferably 1 to 3, more preferably 1 to 2, and still more preferably 1. Furthermore, the aromatic ring in the aromatic compound residue represented by Ar may have a substituent other than a halogen atom. The substituent is not specifically limited so long as it does not interfere the reaction in the condition for production of the invention explained below and does not induce any side reaction, and preferable examples include branched or non-branched alkyl groups having 1 to 12 carbon atoms. The above-mentioned substituents may further be substituted where possible, and may have a ring structure.

Examples of the above-mentioned substituent other than a halogen atom include alkyl group, alkenyl group, aryl group, arylalkyl group, alkylaryl group, arylalkenyl group, alkenylaryl group, aryloxy group, alkoxy group, alkoxyalkyl group, alkoxyaryl group, aryloxyalkyl group, alkylthio group, arylthio group, acyloxy group, alkylsulfoxy group, arylsulfoxy group, acylthio group, formyl group, acyl group, acyloxy group, alkoxycarbonyl group, arylcarbonyl group, arylcarbonyloxy group, aryloxycarbonyl group, cyano group and nitro group.

Specific examples of the alkyl group in these substituents include methyl group, ethyl group, propyl group, butyl group, pentyl group, hexyl group, heptyl group, octyl group, isopropyl group, isobutyl group, s-butyl group, t-butyl group, isopentyl group, neopentyl group, 1-methylbutyl group, isohexyl group, 2-ethylhexyl group, 2-methylhexyl group, cyclopentyl group and cyclohexyl. Specific examples of the aryl group include phenyl group, biphenyl group, naphthyl group, tolyl group, xylyl group, mesityl group, cumenyl group, chlorophenyl group, bromophenyl group, chloromethylphenyl group, methoxyphenyl group, ethoxyphenyl group, phenoxyphenyl group, acetoxyphenyl group, benzoyloxyphenyl group, methylthiophenyl group, phenylthiophenyl group, acetylaminophenyl group, carboxyphenyl group, methoxycarbonylphenyl group, ethoxyphenylcarbonyl group, phenoxycarbonylphenyl group, phenyl group and cyanophenyl group. Examples of the alkenyl group include vinyl group, 1-propenyl group, 1-butenyl group, cynnamyl group and 2-chloro-1-ethenyl group. Examples of the G1 in the acyl group (G1CO-) include hydrogen, and the above-mentioned alkyl groups and aryl groups. These substituents may be further substituted, and examples of the substituents therefore include the above-mentioned substituents and halogen atoms (e.g., chlorine, bromine, fluorine, iodine).

Among the substituents other than a halogen atom, preferable examples include alkyl group, halogenated alkyl group, aryl group, alkenyl group, arylalkyl group, alkylaryl group, aryl alkenyl group, alkenylaryl group, alkoxy group, alkoxy alkyl group, aryloxy group, aryloxyalkyl group, arylthio group, acyloxy group, acyl group, alkoxycarbonyl group, arylcarbonyl group, aryl carbonyloxy group and aryloxycarbonyl group.
Among these substituents, more preferable examples include alkyl group (methyl group, trifluoromethyl group, ethyl group, trifluoroethyl group, propyl group, butyl group, isopropyl group, t-butyl group, t-octyl group), aryl group (phenyl group, tolyl group, mesityl group, cumenyl group, chlorophenyl group, methoxy phenyl group, ethoxy phenyl group, acetoxyphenyl group, benzoyloxyphenyl group), alkoxy group (methoxy group, ethoxy group, isopropoxy group and aryloxy group (phenoxy group).

A plurality of each of Ar, X and n on the sulfonium compound represented by the general formula (1) may be the same or different. Furthermore, the three aromatic compound residues each having n halogen atoms as substituents ((X)n-Ar-) may be the same or different. Preferably, the three residues are the same.

Specific preferable examples of the aromatic compound residue having n halogen atoms as substituents ((X)n-Ar-) in the general formula (1) include groups obtained by removing one hydrogen atom on the aromatic ring from a compound selected from the group consisting of fluorobenzene, chlorobenzene, bromobenzene, iodobenzene, 1,2-difluorobenzene, 1-chloro-2-fluorobenzene, 1,2-dichlorobenzene, 1-bromo-2-chlorobenzene, 2-fluorotoluene, 2-chlorotoluene, 2-bromotoluene, 1-bromo-2-ethylbenzene, 1,3-difluorobenzene, 1,3-dichlorobenzene, 1,3-dibromobenzene, 1,4-difluorobenzene, 1,4-dichlorobenzene, 1,4-dibromobenzene, 3-fluorotoluene, 3-chlorotoluene, 3-bromotoluene, 4-fluorotoluene, 4-chlorotoluene, 4-bromotoluene, 1,3,5-trichlorobenzene, 1-chloronaphthalene, 1-bromonaphthalene, 9-chloroanthracene and 8-bromoanthracene, and more specific and preferable examples include groups obtained by removing one hydrogen atom that is bonded to the carbon atom having the highest electron density among the carbon atoms to which hydrogen atoms are bonded in the aromatic ring of the above-mentioned compounds.

Z represents a counteranion other than a halide ion. Z may be monovalent anion or divalent anion, and preferable examples include sulfonic acid anion, carboxylic acid anion, phosphoric acid anion, boric acid anion and antimonic acid anion, in view of stability and corrosion property to aluminum of the sulfonium compound represented by the general formula (1), and more preferable examples include sulfonic acid anion, phosphoric acid anion and boric acid anion.
Examples of the sulfonic acid anion include alkyl sulfonic acid anions such as methanesulfonic acid anion, halogenated alkyl sulfonic acid anions such as trifluoromethane sulfonic acid anion, aromatic sulfonic acid anions such as 1-naphthalenesulfonic acid anion, and halogenated sulfonic acid anions such as pentafluorobenzenesulfonic acid anion. Furthermore, the alkyl group in the alkyl sulfonic acid anion and the aromatic ring in the aromatic sulfonic acid anion may have a substituent other than sulfonic acid. Examples of the carboxylic acid anion include trifluoroacetic acid anion, benzoic acid anion, 4-methoxybenzoic acid anion, benzoylformic acid anion, acetylformic acid anion and cyclohexanecarboxylic acid anion. Examples of the phosphoric acid anion include hexafluorophosphate anion. Examples of the boric acid anion include tetrafluoroboric acid anion and tetrakis(pentafluorophenyl)boric acid anion. Examples of the antimonic acid anion include hexafluoroantimonic acid anion.

Among the above-explained counteranions, a suitable counteranion may be selected in view of stability, reactivity, and performance as an acid generating agent for resist, a photocation polymerization initiator or a photoradical polymerization initiator of the objective sulfonium compound. Hereinafter representative examples of the sulfonium compound represented by the general formula (1) are shown as preferable exemplary embodiments of the invention, but the invention is not limited to them.

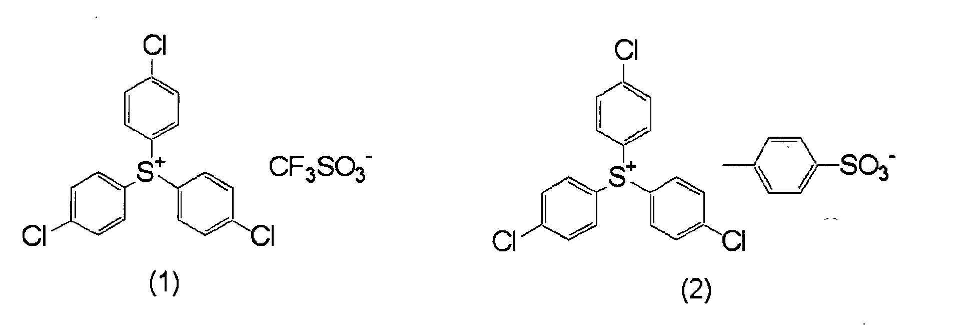

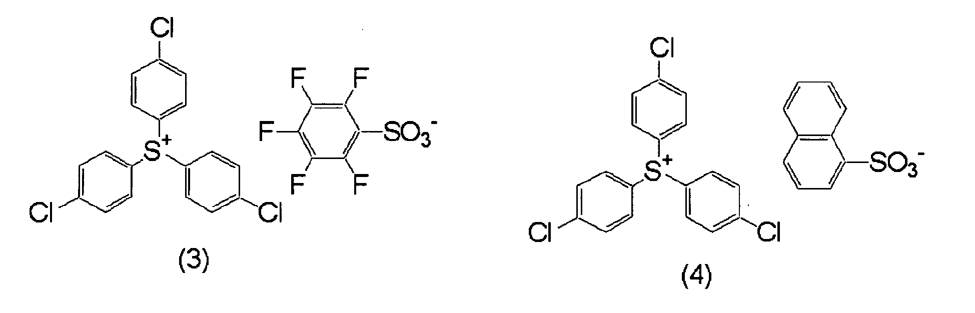

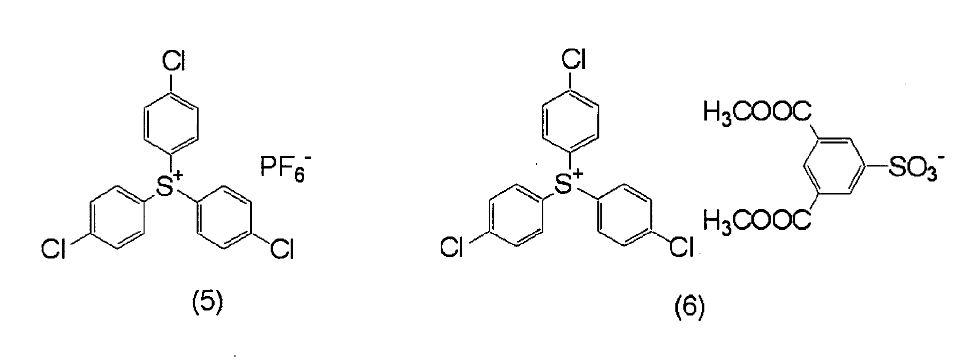

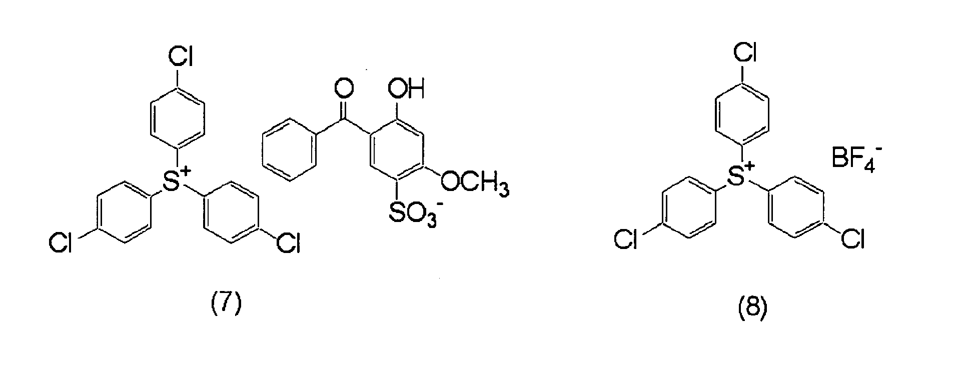

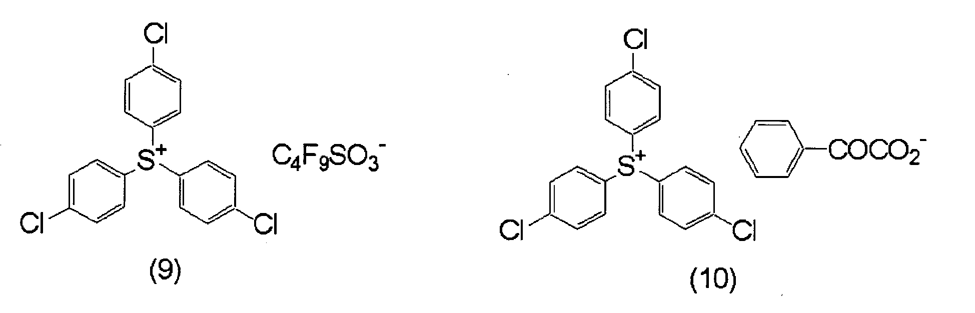

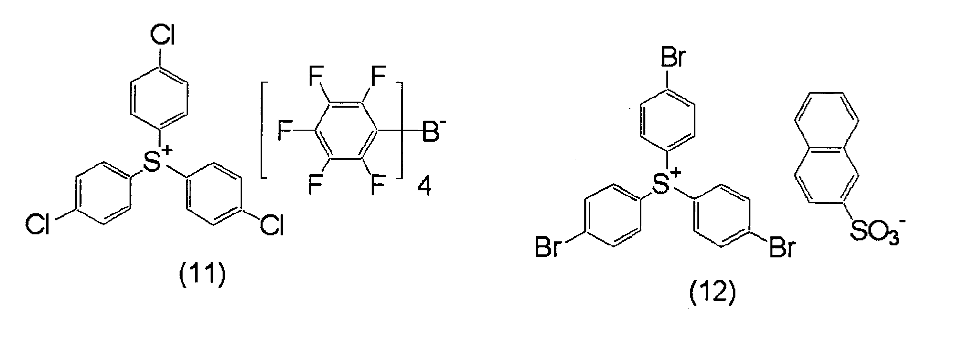

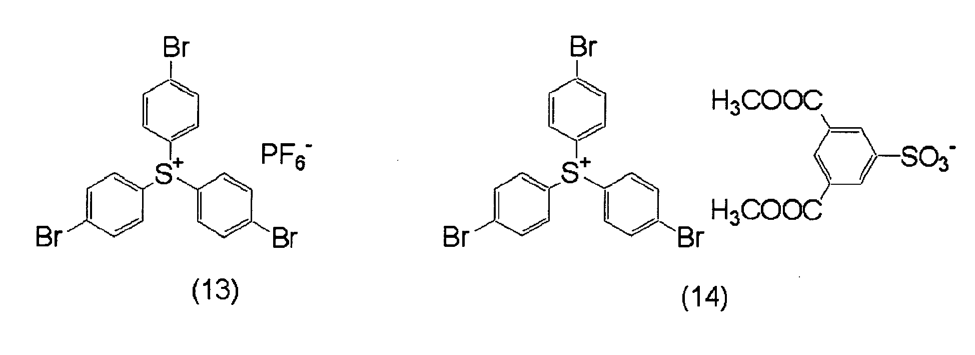

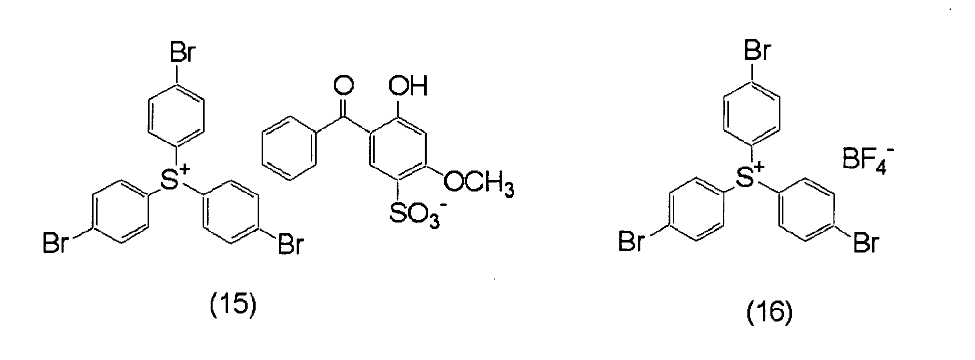

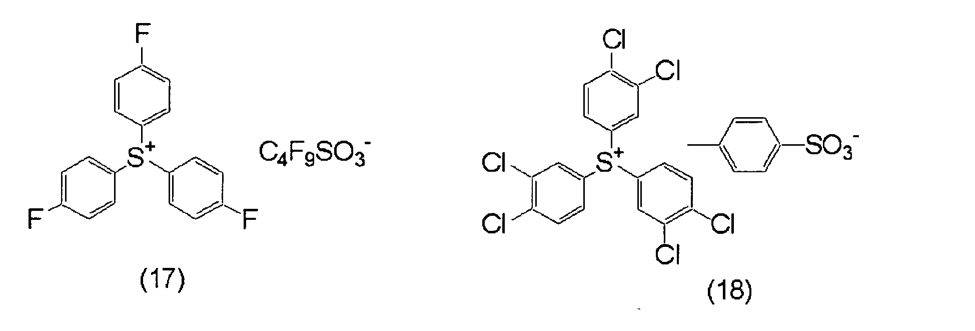

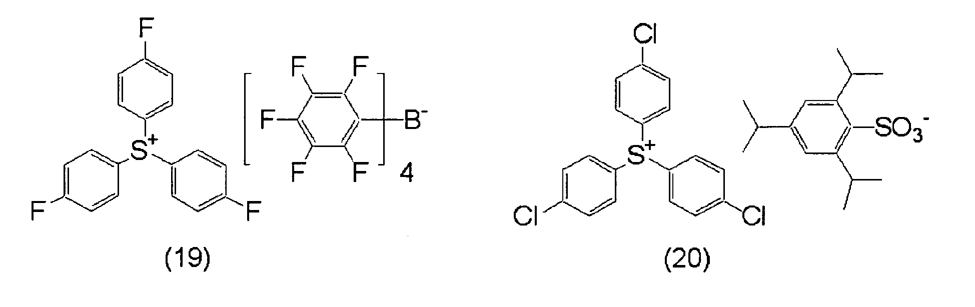

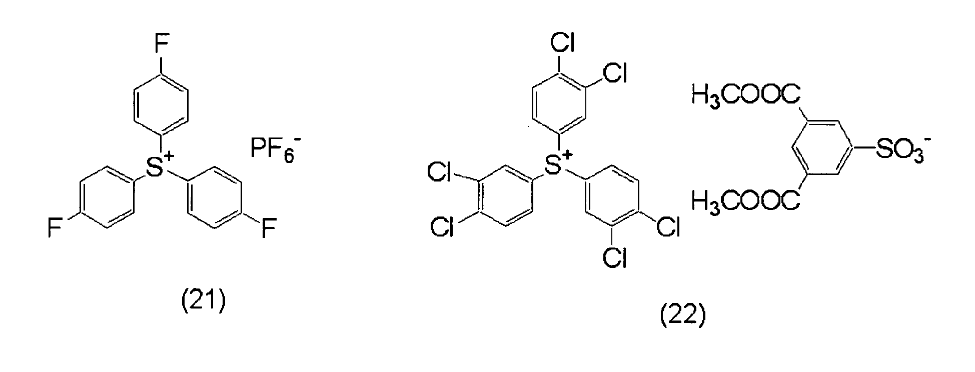

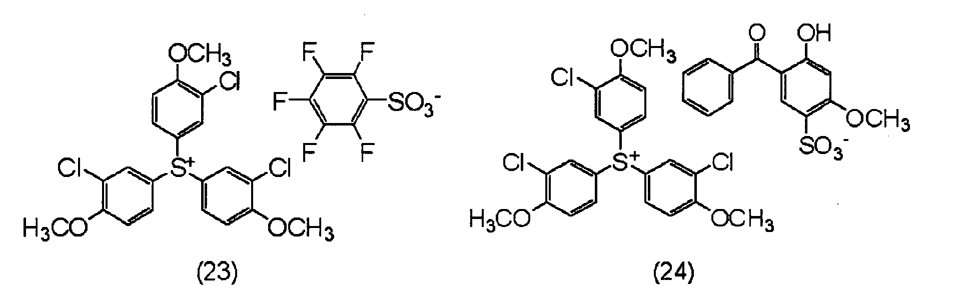

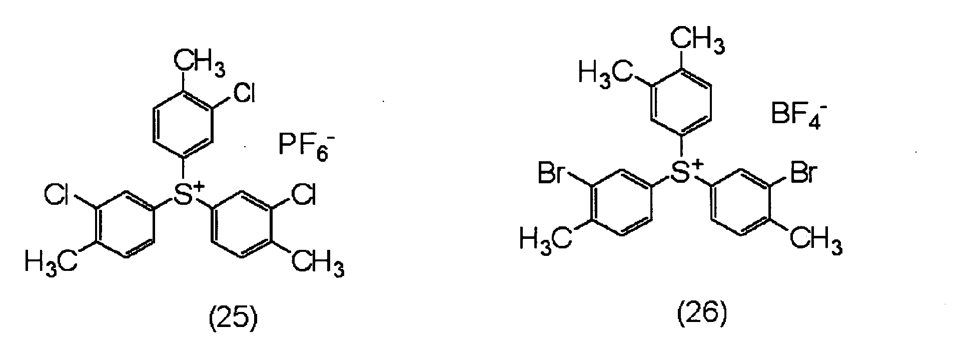

Next, the compound represented by the general formula (2) is explained.

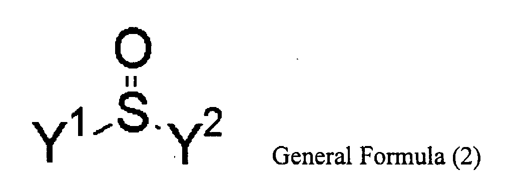

In the general formula (2), Y¹ and Y² are each independently a halogen atom, an alkoxy group or an aryloxy group. Examples of the halogen atom include fluorine atom, chlorine atom, bromine atom and iodine atom. Examples of the alkoxy group include methoxy group, ethoxy group, propoxy group and butoxy group. Examples of the aryloxy group include phenoxy group. Y¹ and Y² may be linked to each other to form a ring structure. Among these, preferable examples of Y¹ and Y² are chlorine atom, bromine atom, methoxy group and ethoxy group, and chlorine atom is the most preferable, in view of reactivity, availability and cost.

Namely, representative examples of the compound represented by the general formula (2) include halogenated thionyls and dialkyl sulfites. Examples of specific compounds that are generally used include dimethyl sulfite, diethyl sulfite, thionyl chloride and thionyl bromide, dimethyl sulfite and thionyl chloride are more preferable, and thionyl chloride is further more preferable.

Next, the compound represented by the general formula (3) is explained.

**M-Z** General Formula (3)

In the general formula (3), Z is as defined above, and preferable specific examples thereof include the same moieties as the anion moieties in the structure exemplified as representative examples of the sulfonium compound represented by the general formula (1). M represents a proton or an alkali metal cation. When M is an alkali metal cation, specific examples include lithium ion, sodium ion, potassium ion, rubidium ion and cesium ion. Among these, the compound represented by the general formula (3) may be suitably selected according to availability and cost of the raw materials, and preferable examples of M are proton, lithium ion, sodium ion and potassium ion, and more preferable examples are proton, sodium ion and potassium ion. Hereinafter representative examples of the compound represented by the general formula (3) are shown as preferable exemplary embodiments of the invention, but the invention is not limited to them. These compounds may be hydrates, and use of hydrates is also encompassed in the invention.

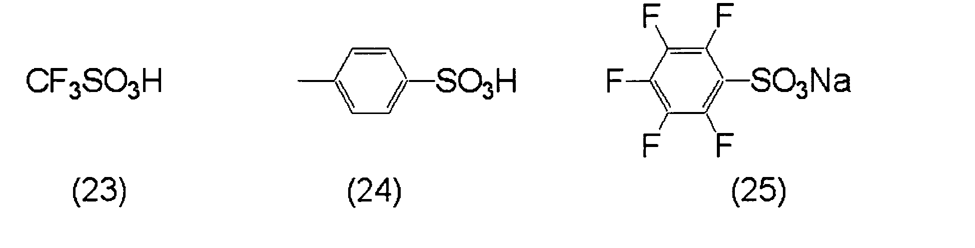

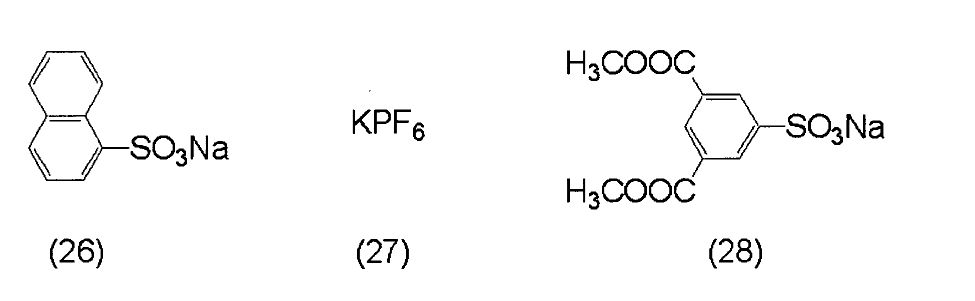

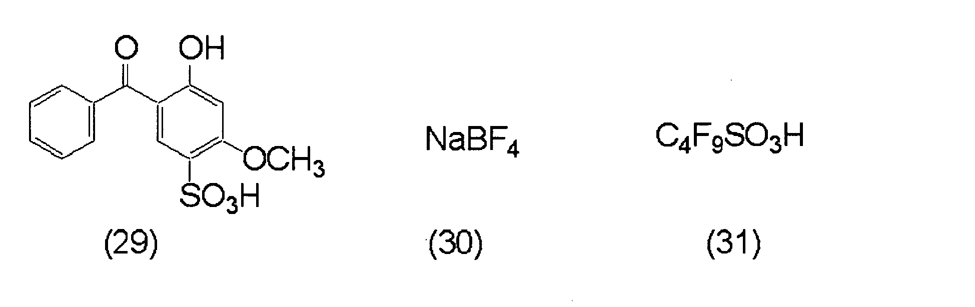

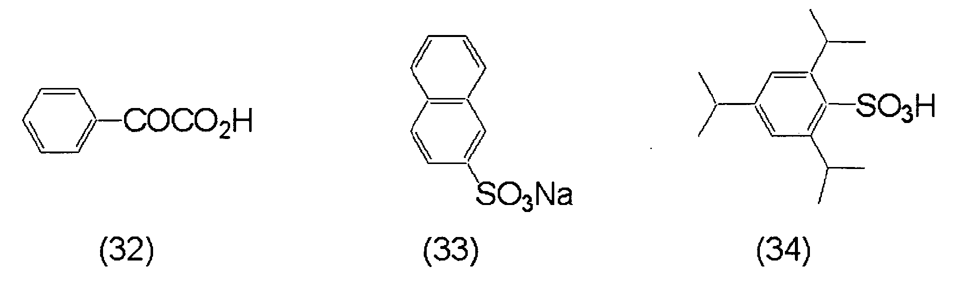

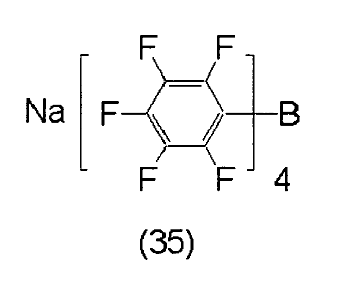

Hereinafter the conditions in the production method of the invention are explained in detail. The first step of the production method of the invention is a step of reacting an aryl halide compound with the compound represented by the general formula (2) in the presence of a Lewis acid. As the Lewis acid, those described in Hisashi Yamamoto, Lewis Acid Reagents: A Practical Approach, The Practical Approach in Chemistry Series, Oxford University Press 1999; Ryoji Noyori et al. ed., Lectures in Graduate School, Organic Chemistry II., Organic Synthesis Chemistry and Bioorganic Chemistry, Tokyo Kagaku Dojin, 1998, and the like may be used, and preferable examples include zinc chloride, aluminum chloride, aluminum bromide, antimony(V) chloride, iron(II) chloride, iron(III) chloride, titanium(IV) chloride, boron trifluoride, tin(IV) chloride and bismuth(III) chloride. Among these, aluminum chloride and aluminum bromide are more preferable, and aluminum chloride is specifically preferable.

The amount of the Lewis acid to be used in the production method of the invention may be in the range of 1 to 10-fold mol relative to the compound represented by the general formula (2), and use of large excess amount does not significantly affect improvement of the yield and production rate of the objective compound. Use of the Lewis acid in a too excess amount makes the subsequent after-treatment and removing steps complicated, which leads to increase in cost, decrease in productivity and increase of waste products and may interfere production on an industrial scale. On the other hand, when the amount of the Lewis acid to be used is less than the range, the yield may sometimes be decreased. Preferable amount of the Lewis acid in the invention is in the range of 2 to 6-fold mol, more preferably in the range of 2.5 to 5-fold mol, further more preferably in the range of 3 to 4-fold mol. The Lewis acid may be used at one time at the beginning, or for example, at two times by 2-fold mol.

The amount of the aryl halide compound to be used is preferably in the range of 3 to 100-fold mol, more preferably in the range of 3 to 50-fold mol, relative to the compound represented by the general formula (2).

A solvent may be used in the step of reacting an aryl halide compound and the compound represented by the general formula (2) in the presence of Lewis acid. The reaction solvent that may be used is not specifically limited so long as it does not cause problems in the operations in the steps, such as failure in stirring due to precipitation of a reaction substrate, a reaction intermediate, a reaction product or the like, does not interfere proceeding of the reaction, and does not cause side reaction, and examples include ether solvents such as 1,2-dimethoxy ethane and tetrahydrofuran, aliphatic chlorine solvents such as dichloromethane and 1,2-dichloroethane, organic acid anhydrides such as acetic anhydride, and aprotic polar solvents such as acetonitrile and sulfolane. These solvents may be used solely or as a combination of two kinds or more. Alternatively, the aryl halide compound that is involved in the reaction as it is may be used as a solvent. Among the above-exemplified methods, a preferable exemplary embodiment of the invention is a method using the aryl halide compound as it is as a solvent.

Although the order of addition of the raw materials is not specifically limited in the step of reacting an aryl halide compound with the compound represented by the general formula (2) in the presence of a Lewis acid, it is preferable to charge an aryl halide compound and/or a Lewis acid, and optionally a solvent, and gradually add the compound represented by the general formula (2) to the mixture.

The reaction temperature in the step of reacting an aryl halide compound with the compound represented by the general formula (2) in the presence of a Lewis acid is generally in the range of -30 to 200°C, preferably in the range of 0 to 180°C, more preferably in the range of 10 to 170°C. The reaction time differs according to the amount to be charged and the reaction temperature, and is generally in the range of 0.5 to 20 hr, more preferably in the range of 3 to 10 hr. Furthermore, in the reaction step, the aryl halide compound that reacts thirdly with the compound represented by the general formula (2) has lower reactivity than those of the aryl halide compounds that react firstly and secondly, due to the effect of steric hindrance and the like. Accordingly, in the above-mentioned reaction step, it is preferable to set the reaction temperature at low reaction temperature at the first stage of the reaction and at high reaction temperature at the later stage of the reaction. Specifically, the reaction temperature at the first stage of the reaction is set preferably in the range of -30 to 40°C, more preferably in the range of -10 to 30°C, and further more preferably in the range of 0 to 25°C. The reaction temperature at the later stage of the reaction is preferably set in the range of 80 to 200°C, more preferably in the range of 100 to 180°C, and further more preferably in the range of 120 to 170°C. The reaction temperature at the later stage of the reaction may be a reflux temperature of the aryl halide compound. Although inert atmosphere is not specifically required in the reaction step, the reaction may be carried out under argon or nitrogen flow.

An after-treatment of the step of reacting an aryl halide compound with the compound represented by the general formula (2) in the presence of a Lewis acid is carried out by cooling the reaction mixture and terminating the reaction by water. Since heat is generated during terminating of the reaction, it is desirable to cool the reaction mixture to 50°C or less, preferably 30°C or less prior to terminating the reaction. Examples of the method of terminating the reaction by water include a method comprising adding a reaction mixture to water, and a method comprising adding water to the reaction mixture, and either of the methods may be applied to the production method of the invention and may be suitably selected according to the circumstances of the production facilities. The water used for terminating of the reaction is preferably ion exchanged water or distilled water, and the amount of water to be used is in the range of 2 to 30-fold volume, preferably in the range of 3 to 10-fold volume relative to the volume used in the step of reacting an aryl halide compound with the compound represented by the general formula (2) in the presence of a Lewis acid. The reaction mixture obtained after terminating the reaction with water is stirred in the range of 10 to 70°C for 30 min to 5 hr and stood still, whereby the mixture is separated into two layers, and the water layer is isolated therefrom. For example, where thionyl chloride is used as the compound represented by the general formula (2) and aluminum chloride is used as a Lewis acid, the sulfonium salt compound generated by the reaction transfers to the water layer.

Next, an extraction treatment of the isolated water layer is carried out with an organic solvent. By this operation, the residual aryl halide compound derived from the above-mentioned reaction step, the sulfoxide form (reaction intermediate), other stained components, and the like are effectively removed by extraction. On the other hand, the objective sulfonium salt compound is dissolved in the water layer. Examples of the organic solvent that may be used for the invention include ether solvents such as diethyl ether, diisopropyl ether, methyl t-butyl ether and methoxybenzene, ester solvents such as ethyl acetate, aliphatic hydrocarbon solvents such as hexane and heptane, and aromatic hydrocarbon solvents, and aromatic hydrocarbon solvents or ester solvents are preferable in view of suitability for mass production on an industrial scale, availability, and easiness of collection and recycling. In the invention, specific examples of the organic solvent that is preferably used include toluene, xylene (which may be o-form, m-form, p-form or mixture thereof in any ratio), mesitylene, isopropylbenzene (cumene), ethyl acetate and n-butyl acetate, of which toluene, xylene, mesitylene and ethyl acetate are more preferable, and toluene and ethyl acetate are further more preferable solvents.

Finally, the compound represented by the general formula (3) is added to the water layer after extraction treatment to exchange the counteranion, whereby the objective sulfonium salt compound represented by the general formula (1) is precipitated. The compound represented by the general formula (3) may be directly used as powder or liquid, or as an aqueous solution having a suitable concentration. The amount of the compound represented by the general formula (3) to be used may be in the range of 0.9 to 3.0-fold mol relative to the theoretical amount of the generated sulfonium salt compound, i.e., the amount of the compound represented by the general formula (2); however, where the amount used is in excess of the above-mentioned range, the yield of the objective product is not significantly affected. In the invention, the amount of the compound represented by the general formula (3) is preferably in the range of 0.8 to 2.5-fold mol, more preferably in the range of 0.9 to 2.0-fold mol, and further more preferably in the range of 1.0 to 1.4-fold mol. Since the objective sulfonium salt compound represented by the general formula (1) has relatively lower solubility in water than those of halogenated products and has good crystallinity, it is precipitated as crystals in many cases. In order to precipitate the objective product, the temperature of the reaction mixture may be decreased, and which is one of the preferable exemplary embodiments of the invention. The precipitated sulfonium salt compound represented by the general formula (1) may be readily isolated by conventional solid-liquid separation and subsequent washing with water and drying.

The sulfonium salt compound represented by the general formula (1) obtained as above generally has so high purity that it may be used as an acid generating agent for resists, a photocation polymerization initiator, or a photoradical polymerization initiator, without further purification. Where necessary, it may be purified by applying a means routinely used in chemical engineering such as washing, crystallization and extraction.

Hereinafter the invention is explained in more detail with referring the Examples and Comparative Examples. However, the invention is not limited to these Examples.

### EXAMPLES

The purities of the sulfonium salts shown in the following Examples were obtained in accordance with area percentages by high performance liquid chromatography (HPLC) under the following measurement conditions.
Measurement conditions:
   Column: Mightysil RP-18GP 150-4.5 (5 µm)
   Column temperature: 25°C
   Eluant: acetonitrile/water = 400/600 (vol/vol), containing triethylamine and phosphoric acid (each 0.1 vol%)
   Elution rate: 1.5 ml/min
   Detection wavelength: 254 nm

### Example 1: Synthesis of the compound (5)

To a mixture consisting of chlorobenzene (30 mL) and anhydrous aluminum chloride (10 g) was added dropwise a mixed liquid of thionyl chloride (4.4 g) and chlorobenzene (10 ml) under nitrogen flow at an internal temperature of 25°C or less. The reaction mixture was stirred at 20 to 25°C for 1 hr, anhydrous aluminum chloride (7 g) was added thereto, and the reaction mixture was stirred at 120 to 150°C for 4 hr. The reaction mixture was cooled to 30°C or less and poured into ion exchanged water (700 mL), and the mixture was stirred at 60°C for 1 hr. Thereafter, from the reaction mixture separated into two layers, the water layer was isolated. The water layer was extraction-treated twice with toluene (100 ml), and the toluene layer was discarded. KPF₆ (7 g) was added to the water layer, and the mixture was stirred for 60 min. The precipitated crystals were collected by filtration, washed with ion exchanged water and isopropanol, and dried to give 16 g of the compound (5) as colorless crystals. Yield 85%. Melting point 170°C, HPLC purity 95%.

### Example 2: Synthesis of the compound (6)

To a mixture consisting of chlorobenzene (100 mL) and anhydrous aluminum chloride (25 g) was added dropwise thionyl chloride (11 g) at an internal temperature of 25°C or less under nitrogen flow. The reaction mixture is stirred at 20 to 25°C for 1 hr, anhydrous aluminum chloride (18 g) was added thereto, and the reaction mixture was stirred at 120 to 150°C for 4 hr. The reaction mixture was cooled to 30°C or less and poured into ion exchanged water (1200 mL), and the mixture was stirred at 60°C for 0.5 hr. Thereafter, from the reaction mixture separated into two layers, the water layer was isolated. The water layer was extraction-treated twice with ethyl acetate (125 ml), and the ethyl acetate layer was discarded. To the water layer was added sodium 3,5-di(methoxycarbonyl)benzenesulfonate (27.3 g), and the mixture was stirred for 5 hr. The precipitated crystals were collected by filtration, washed with ion exchanged water, and dried to give 51 g of the compound (6) as white crystals. Yield 86%. Melting point 69°C, HPLC purity 99% (total of 3,5-di(methoxycarbonyl)benzenesulfonic acid anion moiety and tris(4-chlorophenyl)sulfonium cation moiety).

### Example 3: Synthesis of the compound (7)

To a mixture consisting of chlorobenzene (150 mL) and anhydrous aluminum chloride (40 g) was added dropwise thionyl chloride (17.6 g) under nitrogen flow at an internal temperature of 25°C or less. The reaction mixture was stirred at 20 to 25°C for 1 hr, anhydrous aluminum chloride (30 g) was added thereto, and the reaction mixture was stirred at 120 to 150°C for 4 hr. The reaction mixture was cooled to 30°C or less and poured into ion exchanged water (2000 mL), and the mixture was stirred at 60°C for 1 hr. Thereafter, from the reaction mixture separated into two layers, the water layer was isolated. The water layer was extraction-treated twice with ethyl acetate (200 ml), and the ethyl acetate layer was discarded. To the water layer was added 2-hydroxy-4-methoxybenzophenone-5-sulfonic acid dihydrate (50.8 g), and the mixture was stirred for 4 hr. The precipitated crystals were collected by filtration, washed with ion exchanged water, and dried to give 79 g of the compound (7) as pale yellow crystals. Yield 79%. Melting point 71 to 73°C, HPLC purity 99% (total of 2-hydroxy-4-methoxybenzophenone-5-sulfonic acid anion moiety and tris(4-chlorophenyl)sulfonium cation moiety).

Examples 4 to 7: Synthesis of compounds (3), (17), (21) and (22) Compounds (3), (17), (21) and (22) were synthesized according to methods similar to those in Examples 1 to 3. The results are shown in the following Table.

**Table 1**

| Examples | Compound | Yield (%) | HPLC purity (%) |
|---|---|---|---|
| 4 | 3 | 78 | 99 |
| 5 | 17 | 75 | 99 |
| 6 | 21 | 68 | 99 |
| 7 | 22 | 60 | 90 |

| | | | |
|---|---|---|---|
| *Total of the anion moiety and the sulfonium cation moiety | | | |

## Claims

1. A production method of a sulfonium salt compound represented by the following general formula (1): 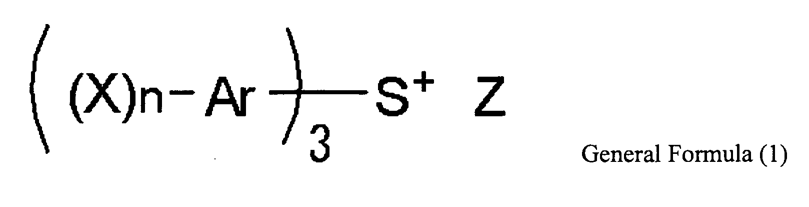 wherein Ar represents an aromatic compound residue, X represents a halogen atom, which is a substituent on the aromatic compound residue, n represents an integer of from 1 to 7, a plurality of each of Ar, X and n may be the same or different, and Z represents a counteranion other than a halide ion,
the method comprising:
reacting an aryl halide compound with a compound represented by the following general formula (2) in the presence of a Lewis acid 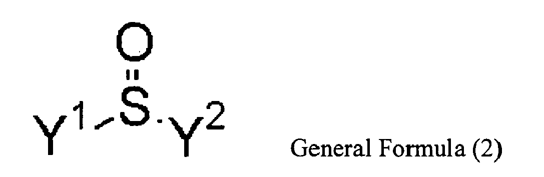 wherein Y¹ and Y² each independently represent a halogen atom, an alkoxy group or an aryloxy group;
terminating the reaction by water in an after-treatment step of the reaction;
isolating a water layer from the reaction mixture that has been separated into two layers;
extraction-treating the water layer by an organic solvent to remove a by-product;
adding a compound represented by the following general formula (3) to the extraction-treated water layer to precipitate the sulfonium salt compound represented by the general formula (1)
**M-Z** General Formula (3)
wherein Z is as defined above, and M represents a proton or an alkali metal cation; and
isolating the precipitated sulfonium salt compound by solid-liquid separation.

2. The production method of claim 1, wherein Ar in the general formula (1) is a benzene ring residue which may further have a substituent other than X (wherein X is as defined in claim 1).

3. The production method of claim 1 or 2, wherein Y¹ and Y² in the general formula (2) are each independently a halogen atom, a methoxy group or an ethoxy group.

4. The production method of any one of claims 1 to 3, wherein the Lewis acid is selected from the group consisting of zinc chloride, aluminum chloride, aluminum bromide, antimony (V) chloride, iron (II) chloride, iron (III) chloride, titanium (IV) chloride, boron trifluoride, tin (IV) chloride and bismuth (III) chloride.

5. The production method of any one of claims 1 to 4, wherein the organic solvent for extraction-treating the water layer comprises an organic solvent selected from the group consisting of an aromatic hydrocarbon solvent, an aliphatic hydrocarbon solvent, an ether solvent and an ester solvent.

6. The production method of any one of claims 1 to 5, wherein the precipitated sulfonium salt compound is crystals.
